(19) **Europäisches Patentamt / European Patent Office / Office européen des brevets**

(11) **EP 1 624 795 B1**

(12) **EUROPÄISCHE PATENTSCHRIFT**

(45) Veröffentlichungstag und Bekanntmachung des Hinweises auf die Patenterteilung:
**22.08.2012 Patentblatt 2012/34**

(21) Anmeldenummer: **04739203.0**

(22) Anmeldetag: **14.05.2004**

(51) Int Cl.:
*A61B 3/10* *(2006.01)*    *G01B 11/14* *(2006.01)*

(86) Internationale Anmeldenummer:
**PCT/EP2004/005170**

(87) Internationale Veröffentlichungsnummer:
**WO 2004/103169 (02.12.2004 Gazette 2004/49)**

(54) **VERFAHREN UND ANORDNUNG ZUM VERMESSEN DES VORDEREN AUGENABSCHNITTS**

METHOD AND ARRANGEMENT FOR MEASURING THE FRONT SECTION OF THE EYE

PROCEDE ET DISPOSITIF PERMETTANT DE MESURER LA SECTION OCULAIRE ANTERIEURE

(84) Benannte Vertragsstaaten:
**AT BE BG CH CY CZ DE DK EE ES FI FR GB GR HU IE IT LI LU MC NL PL PT RO SE SI SK TR**

(30) Priorität: **22.05.2003 DE 10323920**

(43) Veröffentlichungstag der Anmeldung:
**15.02.2006 Patentblatt 2006/07**

(60) Teilanmeldung:
**12157249.9 / 2 465 412**

(73) Patentinhaber: **Carl Zeiss Meditec AG**
**07745 Jena (DE)**

(72) Erfinder:
 • **BARTH, Roland**
   **07743 Jena (DE)**
 • **BERGNER, Roland**
   **07745 Jena (DE)**
 • **VOIGT, Klaus-Ditmar**
   **07745 Jena (DE)**
 • **BEHRENDT, Frank**
   **07743 Jena (DE)**
 • **DIETZEL, Burkhard**
   **07616 Bürgel (DE)**
 • **HOFMANN, Eberhard**
   **07646 Bollberg (DE)**
 • **STOBER, Gert**
   **07743 Jena (DE)**
 • **KLOPFLEISCH, Peter**
   **07743 Jena (DE)**

(56) Entgegenhaltungen:
   **DE-A- 4 446 183    DE-A- 10 108 797**
   **US-A- 5 474 548    US-A- 6 082 859**

 • **DREXLER W ET AL: "Submicrometer precision biometry of the anterior segment of the human eye." INVESTIGATIVE OPHTHALMOLOGY & VISUAL SCIENCE. UNITED STATES JUN 1997, Bd. 38, Nr. 7, Juni 1997 (1997-06), Seiten 1304-1313, XP009035980 ISSN: 0146-0404 in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die Erfindung betrifft ein Verfahren und eine Anordnung zum Vermessen des vorderen Augenabschnitts. Sie ist insbesondere anwendbar zur Bestimmung der Parameter, die zur Auswahl einer geeigneten Intraokularlinse für eine Kataraktoperation benötigt werden. Außerdem kann sie auch zur Qualitätskontrolle nach erfolgter Implantation einer Intraokularlinse benutzt werden.

Aus DE 198 57 001 ist ein Gerät bekannt, welches zur berührungslosen Messung der Augenlänge, Hornhautkrümmung und Vorderkammertiefe verwendet werden kann. Dabei wird die Achslänge interferometrisch, die Hornhautkrümmung mittels Bildverarbeitung aus Reflexbildern von unter einem bestimmten Winkel auf die Hornhaut projizierten Messmarken und die Vorderkammertiefe aus der Auswertung der Rückstreuung einer spaltförmigen Beleuchtung der Augenlinse bestimmt.

Die beschriebene Messung der Vorderkammertiefe funktioniert bei pseudophaken Augen nicht, da die implantierten Intraokularlinsen (IOL) in der Regel keine streuende Wirkung aufweisen. Die interferometrische Vermessung der Augenachslänge ist bekannt aus "Optical Measurement of the Axial Eye Length by Laser Doppler Interferometry" (Ch. Hitzenberger, Investigative Ophthalmology and Visual Science, Vol. 32, Nr. 3, S. 616, März 1991), auf deren Offenbarung im weiteren Bezug genommen wird.

DE 101 08 797 beschreibt ein Verfahren zur Ermittlung des Durchmessers von Pupille und Iris mit Mitteln der digitalen Bildverarbeitung, wobei unter anderem auch der Winkel zwischen Sehachse und optischer Achse des Auges bestimmt werden kann.

In "Submicrometer Precision Biometry of the Anterior Segment of the Human Eye" (Drexler et all, Investigative Ophthalmology & Visual Science, Vol. 38, Nr. 7, S. 1304, Juni 1997) wird ein Versuchsaufbau beschrieben, mit welchem das vordere Auge interferometrisch vermessen werden kann.

Dabei wird das Auge beim Messvorgang mit einem kollimierten Lichtbündel bestrahlt. Dabei sind die von der Hornhaut und den Linsenflächen reflektierten Lichtanteile, welche auf einen Photodetektor abgebildet werden, relativ schwach.

Das Auge muss für die Messung so ausgerichtet werden, dass seine optische Achse mit der Messachse des Gerätes übereinstimmt. Dazu wird dem Patienten entlang einer feststehenden Achse (koaxial) ein kollimiertes Fixierlicht angeboten, welches für das zu vermessendes Auge über einen Spiegel eingekoppelt wird. Die Einstellung eines Winkels zwischen Sehachse des Patienten und Messachse des Versuchsaufbaus erfolgt mittels eines Scan-Spiegels.

Bereits bei Abweichung der optischen Achse von der Messachse im Bereich von 1° (z.B. bedingt durch Fixationsprobleme bzw. Nystagmus) können sich die Reflexe von Hornhaut und Linse nicht mehr überlagern, sodass kein Interferenzmesssignal entsteht. Die Messung ist damit sehr empfindlich gegenüber Verkippen des Patientenauges. Weiterhin erscheint das Fixierlicht dem Patienten immer im Unendlichen, was sich als nachteilig erweisen kann.

Die Lage der optischen Achse wird gesucht, indem der Scan-Spiegel in zwei zueinander orthogonalen Richtungen solange verkippt wird, bis alle Messsignale von Hornhaut und Linse zeitgleich nachzuweisen sind. Dieses Verfahren ist extrem zeitaufwendig und führt auch nicht bei allen Patienten zum gewünschten Erfolg. Für den Einsatz in der täglichen klinischen Routine ist dieses Verfahren zu umständlich.

Die Erfindung setzt sich das Ziel die Nachteile des Standes der Technik zu überwinden und eine schnelle und sichere Vermessung des vorderen Augenabschnitts mit interferometrischen Mitteln zu ermöglichen.

Diese Aufgabe wird erfindungsgemäß durch die Merkmale der unabhängigen Ansprüche gelöst, bevorzugte Weiterbildungen sind in den abhängigen Ansprüchen beschrieben.

Die Erfindung wird im folgenden anhand der Figuren beschrieben.
Es zeigen:

Fig. 1 eine schematische Anordung eines bevorzugten Ausführungsbeispiels
Fig. 2 eine schematische Ansicht eines Teils des Interferometerstrahlengangs
Fig. 3 und 4 zwei (unvollständige) Messergebnisse
Fig. 5 ein vollständiges Messergebnis
Fig. 6 die Transmissionscharakteristik eines der Strahlteiler aus Fig. 1

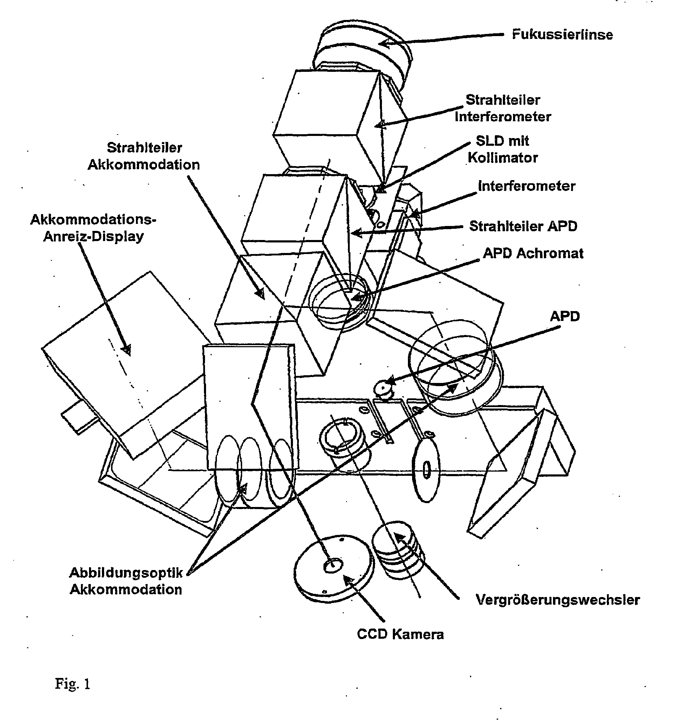

In Fig. 1 wird das von einer SLD (Super-Lumineszenz-Diode) emittierte Licht in bekannter Form über ein Interferometer und einen Strahlteiler in Richtung Patientenauge (hier nicht dargestellt) gelenkt. Zwischen Strahlteiler und Auge befindet sich eine Fokussierlinse, welche den Messstrahl in den vorderen Augenabscbnitt fokussiert.

Das vom Auge reflektierte Licht wird durch die Fokussierlinse annähernd kollimiert und mittels Strahlteiler APD und APD Achromat auf den hinter einer hier nicht dargestellten Blende liegenden Photodetektor APD (Avalanche Photo Detektor) abgebildet Ein geringer Lichtanteil (ca. 5%) wird im Strahlteiler transmittiert und mittels geeigneter Optik (Vergrößerungswechsler) auf eine CCD Kamera abgebildet.

Durch gezieltes Defokussieren des Messstrahles mit Hilfe der Fokussierlinse oder Verschieben der Optik relativ zum Patientenauge entlang der optischen Achse der Anordnung werden die Reflexe von Hornhaut und Linse (jeweils Vorder- und Rückseite) unscharf und vergrößert auf den Photodetektor APD abgebildet. Durch die vergrößerte Abbildung der Reflexe wird ein Überlappen der Reflexe in einem größeren Bereich möglich, dadurch verringert sich die Empfindlichkeit gegenüber Verkippung und seitliche Verschiebung des Auges. Unterschiedlich große Lichtintensitäten der Reflexe auf der APD werden vorteilhafterweise durch entsprechende Anpassung der elektronischen Verstärkung ausgeglichen. Die Verstärkung kann dabei automatisch angepasst oder um einen mittleren konstanten Faktor reduziert werden.

Die Empfindlichkeit der Anordnung auf Abweichungen der Augachse von der optischen Achse der Anordnung hängt außerdem vom Verhältnis der Brennweite der Fokussierlinse zu der Brennweite des APD Achromat (APD Linse) ab. Je größer der Quotient beider Brennweiten wird, umso geringer ist diese Empfindlichkeit, so dass auch größere Abweichungen noch ein auswertbares Signal liefern.

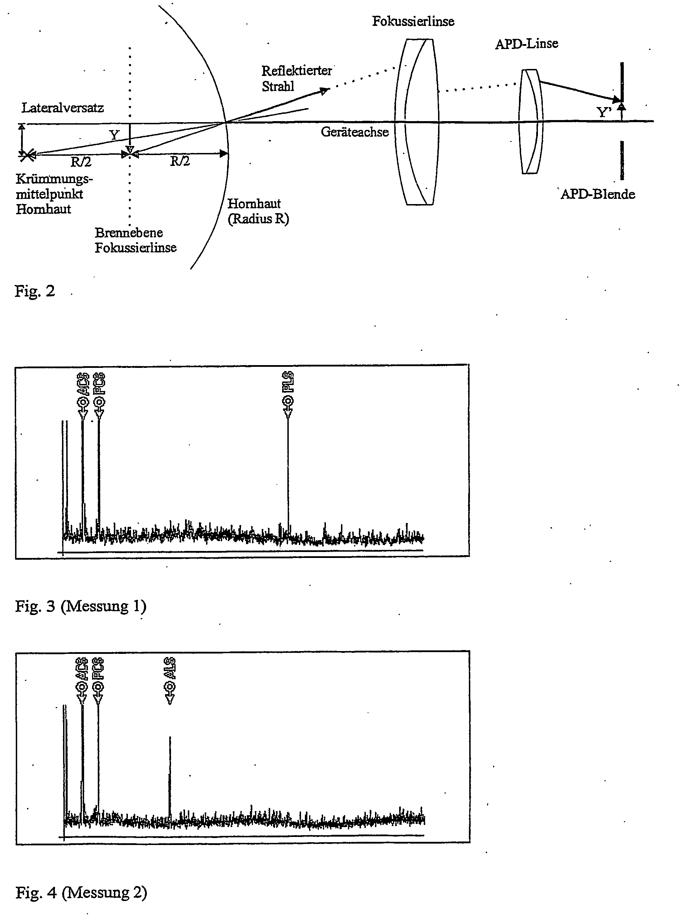

Figur 2 beschreibt diese Abhängigkeit der seitlichen Justiergenauigkeit zum Auge von den Brennweiten der verwendeten Linsen.

Das Auge sei gegenüber der Geräteachse (opt. Achse) lateral versetzt um y.

Der Hauptstrahl muss nach Reflexion an Hornhaut und Durchgang durch Fokussier- und APD-Linse die APD-Blende (Ø=2y') passieren. Die APD-Blende liegt in der Brennebene der APD-Linse.

Zur Ermittlung der Abhängigkeit des zulässigen Lateralversatzes von den Brennweiten der Linsen betrachte man die Abbildung des virtuellen Objektpunktes (Höhe y) in der Brennebene der Fokussierlinse. Für die Abbildung dieses gedachten Objektes auf den Rand der APD-Blende ergibt sich:

$$y' \approx \frac{f_{APD}}{f_{FOK}} y \quad \text{und damit für den Lateralversatz} \quad y \approx \frac{f_{FOK}}{f_{APD}} y'.$$

Wobei:

$f_{FOK}$, $f_{APD}$ : Brennweiten von Fokussier- bzw. APD-Linse
y : zulässiger Lateralversatz
y': halber Blendendurchmesser

Ein optimaler Wert für dieses Verhältnis $f_{FOK}$: $f_{APD}$ liegt etwa zwischen 1,5 und 4.

Für den Fall, dass trotz des vergrößerten Messbereiches nicht alle Signale von Hornhaut und Linse gleichzeitig detektiert werden können, werden mehrere Messungen durchgeführt, deren Ergebnisse geeignet zu einem alle notwendigen Messsignale enthaltenden Gesamtergebnis kombiniert werden.

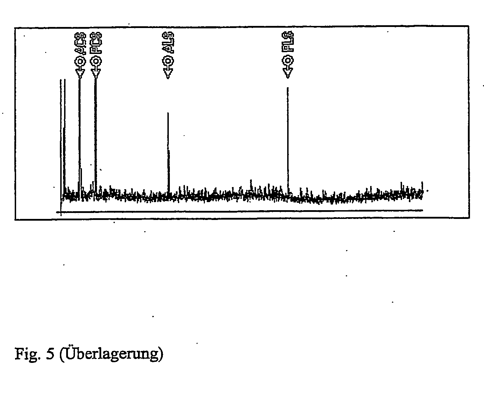

Eine Möglichkeit für ein solches Verfahren ist in den Fig. 3 bis 5 dargestellt.

Es bedeuten:

ACS - anterior cornea surface (Hornhautvorderfläche)
PCS - posterior cornea surface (Hornhautrückfläche)
ALS - anterior lens surface (Linsenvorderfläche)
PLS - posterior lens surface (Linsenrückfläche)

In Fig. 3 ist kein Reflexsignal der Linsenvorderfläche (ALS) detektiert worden, in Fig. 4 fehlt der Reflex der Linsenrückfläche. In einem ersten Schritt werden die beiden Messreihen auf einen in beiden vorhandenen Reflex, welcher vorzugsweise der Reflex von der Hornhautvorderfläche (ACS) ist, transformiert. Anschließend werden die anderen Werte der Reflexsignale aus beiden Messungen in die Gesamtmessung übernommen. Je nach Qualität der Messungen und Erfordernis können auch mehr als zwei Messungen durchgeführt und in der dargestellten Art und Weise miteinander kombiniert werden. Dabei ist es vorteilhaft wenn aus den verschiedenen Messungen jeweils die zu den Reflexen gehörenden Maximalwerte in die Gesamtmessung übernommen werden.

Es ist für die Durchführung der Messungen günstig, wenn die optische Achse der Messanordnung und die optische Achse des Auges zueinander ausgerichtet sind. Der Durchstoßpunkt der Sehachse durch die Pupille markiert sich durch Reflexion einer koaxialen LED welche in der optischen Achse der Messanordnung angebracht ist (in Fig. 1 aus Gründen der Übersichtlichkeit nicht dargestellt), auf welche der Patient fixiert. Mittels des in DE 101 08 797 beschriebenen Verfahrens kann dann die Abweichung der Sehachse von der optischen Achse des Auges bestimmt werden.

Für die Einstellung des zur Messung erforderlichen Winkels wird dann ein in das Gerät integriertes LC-Display (Akkomodations-Anreiz-Display) verwendet, welches über eine Abbildungsoptik Akkomodation sowie einen Strahlteiler Akkomodation in den Strahlengang eingespiegelt wird. Ein Testzeichen (z.B. Kreuz, Punkt o. ä.) wird in Abhängigkeit von dieser bestimmten Abweichung automatisch so verstellt, dass das Patientenauge durch Fixieren auf dieses Zeichen zur Messung ausgerichtet wird. Durch die Fokussierlinse wird die Detektierbarkeit dieser LED entscheidend verbessert, was zur Erhöhung der Messgenauigkeit beiträgt.

Die Einspiegelung der auf dem LC-Display dargestellten Testzeichen im sichtbaren Wellenlängenbereich in den Messstrahlengang (Infrarot) stellt spezifische Anforderungen an den verwendeten Strahlteiler (Strahlteiler Akkommodation).

Hierzu ist eine möglichst hohe Effizienz des einzukoppelnden optischen Signals vom LC-Display in den zum Patientenauge führenden Strahlengang notwendig. Zur Realisierung dieser Forderung wird die Eigenschaft ausgenutzt, dass das vom LC-Display kommende Licht wegen dessen Funktionsprinzips linear polarisiert ist. Der Strahlteiler weist eine nahezu 100%ige Reflexion für s-polarisiertes Licht im VIS-Bereich von 400 bis 650nm auf. Gleichzeitig wird eine möglichst verlustfreie Transmission im nahen-Infrarotbereich (830nm...1000nm) realisiert.

Das Schichtdesign erfüllt diese Forderungen für einen Einfallswinkelbereich um 45°. Die eingesetzten Materialien sind bezüglich Brechzahl Substrat, Kittbrechzahl und der Brechzahl der Beschichtungssubstanzen aufeinander abgestimmt Für diesen speziellen Einsatz wurden folgende Materialien ausgewählt:

Substrat:

BK7 n = 1,64
Kitt n =1,52
H n = 2,30
L n= 1.48

Das Design besteht aus 23 Wechselschichten H L.

Für vergleichbare Teiler können durch eine geeignete Wahl der Brechzahlen von Substrat und Beschichtungssubstanzen sowie des Einfallswinkels entsprechende Bauelemente gefertigt werden. Durch einen Faktor kann das komplette Schichtsystem bezüglich Kantenlage verschoben werden.

Parameter:

hohe Reflexion von 400... 660nm, s-polarisiert
hohe Transmission von 830....1400 nm, unpolarisiert und s-polarisiert

Beispieldaten:

| 1 | Ti02 | 66.28nm |
|---|------|---------|
| 2 | Si02 | 91.88nm |
| 3 | Ti02 | 102.33nm |
| 4 | Si02 | 141.87nm |
| 5 | Ti02 | 75.32nm |
| 6 | Si02 | 156.86nm |
| 7 | Ti02 | 75.32nm |
| 8 | Si02 | 156.86nm |
| 9 | Ti02 | 75.32nm |
| 10 | Si02 | 156.86nm |
| 11 | Ti02 | 77.62nm |
| 12 | Si02 | 130.85nm |
| 13 | Ti02 | 63.37nm |
| 14 | Si02 | 133.9nm |
| 15 | Ti02 | 49.88nm |
| 16 | Si02 | 113.15nm |

(fortgesetzt)

| 17 | Ti02 | 49.88nm |
|----|------|---------|
| 18 | Si02 | 113:15nm |
| 19 | Ti02 | 49.88nm |
| 20 | Si02 | 113.15nm |
| 21 | Ti02 | 64.76nm |
| 22 | Si02 | 118.7nm |
| 23 | Ti02 | 41.83nm |

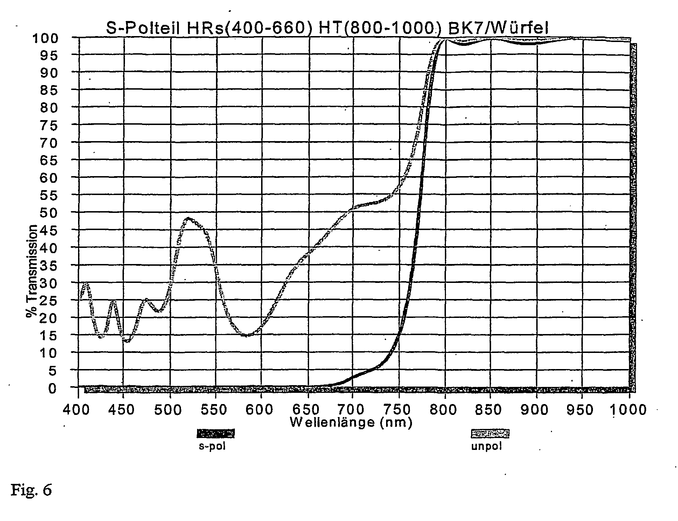

Die mit diesem Schichtaufbau erreichten Transmissionswerte für s-polarisiertes und unpolarisiertes Licht sind in Fig. 6 dargestellt.

Vorteilhaft wird das gleiche Display genutzt, um das Auge zum Akkommodieren zu veranlassen.

Die Abbildungsoptik Akkommodation bildet in einer definierten Lage des Displays Testzeichen nach unendlich ab. Der Patient kann im akkommodationslosen Zustand die dargebotenen Tests betrachten. Durch (z.B. motorische) Verschiebung des Displays senkrecht zu seiner Ausdehnung erfolgt die Testzeichenabbildung in definierte Abstände vor das Patientenauge (z.B. 40 cm). Der Patient kann die Tests nur scharf sehen, wenn er akkommodiert. Wenn in auf diese Weise erreichbaren verschiedenen Akkomodationszuständen eine Messung im vorderen Augenabschnitt erfolgt, kann der jeweilige Abstand der Linsenvorder- und -rückseiten von der Hornhaut bestimmt werden, womit die für die Akkommodation verantwortlichen Bewegungen bzw. Formveränderungen der Augenlinse nachweisbar sind. Dieses ist insbesondere auch zur Kontrolle der Wirksamkeit akkommodierender Intraokularlinsen verwendbar.

Durch Auswertung der Stellung des Displays, in welcher der Patient gerade noch scharf sehen kann, ist es weiterhin möglich, die Akkommodationsbreite zu bestimmen.

Ein weiterer Vorteil der erfindungsgemäßen Lösung ist die Möglichkeit der Anpassung der Messanordnung an eine eventuell vorhandene Fehlsichtigkeit der Patienten durch entsprechende Verschiebung des LC-Displays bis der Patient die dargestellten Testzeichen scharf sieht.

Für bestimmte Anwendungsfälle (z.B. vor Lasik-OP) ist es wichtig, die Hornhäutdicke an mehreren Punkten zu messen. Dazu werden auf dem LC-Display an verschiedenen vorbestimmten Stellen Fixationsreize (z. B. Kreuze, Punkte o. ä.) dargestellt Dadurch wird der Patient veranlasst, entsprechende Blickwendungen zu vollziehen, so dass die anschließenden Messungen der Hornhautdicke jeweils an dem vorbestimmten Punkt durchgeführt werden. Solche vorbestimmten Punkte können neben der Augenachse auch 1,5mm, 3mm und 4,5 mm von der Achse entfernt sein.

Die Realisierung der Erfindung ist nicht an das dargestellte Ausführungsbeispiel gebunden, fachmännisch Weiterentwicklungen sind möglich ohne den Schutzbereich zu verlassen.

**Patentansprüche**

1. Anordnung zum Vermessen des vorderen Augenabschnitts eines Patientenauges, wobei eine Interferometeranordnung zur Messung der Abstände optische Funktionsflächen des Auges und eine Lichtquelle für die Erzeugung eines Fokussierreizes des Patienten vorgesehen ist sowie
   im Strahlengang des Interferometers eine Fokussierlinse vorgesehen ist, welche das Auge mit einem konvergenten Strahlenbündel beleuchtet **gekennzeichnet dadurch dass** die Lichtquelle für die Erzeugung des Fokussierreizes ein Flächenlichtmodulator mit einstellbarer Lichtverteilung ist, wobei der Flächenlichtmodulator eine LCD-Matrix ist oder ein DMD enthält.

2. Anordnung zum Vermessen des vorderen Augenabschnitts nach Anspruch 1, **gekennzeichnet dadurch, dass** Mittel zur Veränderung der Fokussierung des Flächenlichtmodulators bezüglich des Patientenauges vorgesehen sind.

3. Anordnung zum Vermessen des vorderen Augenabschnitts nach Anspruch 1, **gekennzeichnet dadurch, dass** Mittel zur Verschiebung einer Optik der Anordnung relativ zum Patientenauge vorgesehen sind.

4. Anordnung zum Vermessen des vorderen Augenabschnitts nach Anspruch 1, **gekennzeichnet dadurch**, das sich die Brennweite der Fokussierlinse zur Brennweite einer einem Detektor der Interferometeranordnung zugeordneten Linse wie 1,5:1 1 bis 4:1 verhält.

**5.** Verfahren zum Vermessen des vorderen Augenabschnitts mit einer Anordnung nach Anspruch 1, **gekennzeichnet dadurch, dass** mehrere interferometrische Messungen der optischen Grenzflächen des vorderen Augenabschnitts durchgeführt werden, wobei einander entsprechende Messwerte detektiert werden und durch Anpassung auf jeweils in mehr als einer Messung gemessene sich entsprechende Werte ein vollständiger Satz von Messwerten berechnet wird.

**6.** Verfahren zum Vermessen des vorderen Augenabschnitts nach Anspruch 5, **gekennzeichnet dadurch, dass** die relative Lage der Augenachse zur optischen Achse der Anordnung bestimmt wird und durch Anbieten eines entsprechend angepassten Fixationsreizes mittels des Flächenlichtmodulators eine Ausrichtung der beiden Achsen bewirkt wird.

**7.** Verfahren zum Vermessen des vorderen Augenabschnitts, insbesondere der Hornhautdicke, mit einer Anordnung nach Anspruch 1, **gekennzeichnet dadurch, dass** durch definierte Erzeugung von Fokussierreizen unter vorbestimmten Winkeln zur optischen Achse der Interferometermordnung eine Messung an vorbestimmten Punkten der Hornhaut realisiert wird.

**8.** Verfahren zum Vermessen der Akkommodationsbreite mit einer Anordnung nach Anspruch 1 und 2, **gekennzeichnet dadurch, dass** ein Verschiebebereich des Flächenlichtmodulators, in welchem der Patient auf diesem dargestellte Testzeichen scharf sehen kann detektiert wird.

**9.** Verfahren zum Vermessen des vorderen Augenabschnitts mit einer Anordnung nach Anspruch 1 und 2, **gekennzeichnet dadurch, dass** zur Anpassung an eventuelle Fehlsichtigkeiten des Patienten der Flächenlichtmodulator entsprechend verschoben wird.

**Claims**

**1.** Arrangement for measuring the front section of the eye of a patient eye, provision being made for an interferometer arrangement for measuring the distances of optical functional areas of the eye and for a light source for producing a focussing stimulus of the patient and provision also being made for a focussing lens in the beam path of the interferometer, said lens illuminating the eye with a converging bundle of rays, **characterized in that** the light source for producing the focussing stimulus is a spatial light modulator with an adjustable light distribution, the spatial light modulator being an LCD matrix or containing a DMD.

**2.** Arrangement for measuring the front section of the eye according to Claim 1, **characterized in that** means are provided for changing the focussing of the spatial light modulator in respect of the patient eye.

**3.** Arrangement for measuring the front section of the eye according to Claim 1, **characterized in that** means are provided for displacing an optical system of the arrangement relative to the patient eye.

**4.** Arrangement for measuring the front section of the eye according to Claim 1, **characterized in that** the ratio of the focal length of the focussing lens to the focal length of a lens associated with a detector of the interferometer arrangement is between 1.5:1 and 4:1.

**5.** Method for measuring the front section of the eye using an arrangement according to Claim 1, **characterized in that** a plurality of interferometric measurements of the optical boundaries of the front section of the eye are carried out, with mutually corresponding measurement values being detected and a complete set of measurement values being calculated by adaptation to mutually corresponding values, respectively measured in more than one measurement.

**6.** Method for measuring the front section of the eye according to Claim 5, **characterized in that** the relative position of the eye axis with respect to the optical axis of the arrangement is determined and an alignment of the two axes is brought about by offering an appropriately adapted fixation stimulus by means of the spatial light modulator.

**7.** Method for measuring the front section of the eye, more particularly the corneal thickness, using an arrangement according to Claim 1, **characterized in that** a measurement at predetermined points on the cornea is implemented by defined production of focussing stimuli under predetermined angles to the optical axis of the interferometer arrangement.

8. Method for measuring the accommodation range using an arrangement according to Claim 1 or 2, **characterized in that** a displacement region of the spatial light modulator in which the patient can see test symbols situated thereon in focus is detected.

9. Method for measuring the front section of the eye using an arrangement according to Claim 1 or 2, **characterized in that** the spatial light modulator is appropriately displaced for adapting to possible refractive errors of the patient.

**Revendications**

1. Dispositif de mesure du segment antérieur de l'oeil d'un patient, dans lequel un interféromètre est prévu pour mesurer les distances des surfaces optiquement fonctionnelles de l'oeil et une source de lumière est prévue pour générer une impulsion de focalisation du patient, et il est prévu sur le trajet du faisceau de l'interféromètre une lentille de focalisation qui éclaire l'oeil avec un faisceau de rayons convergent, **caractérisé en ce que** la source de lumière destinée à générer l'impulsion de focalisation est un modulateur spatial de lumière ayant une distribution lumineuse réglable, dans lequel le modulateur spatial de lumière est une matrice de cristaux liquides ou contient un DMD.

2. Dispositif de mesure du segment antérieur de l'oeil selon la revendication 1, **caractérisé en ce que** des moyens sont prévus pour modifier l'orientation du modulateur spatial de lumière par rapport à l'oeil du patient.

3. Dispositif de mesure du segment antérieur de l'oeil selon la revendication 1, **caractérisé en ce que** des moyens sont prévus pour décaler une optique du dispositif par rapport à l'oeil du patient.

4. Dispositif de mesure du segment antérieur de l'oeil selon la revendication 1, **caractérisé en ce que** le rapport de la distance focale de la lentille de focalisation à la distance focale d'une lentille associée à un détecteur du dispositif interférométrique est de 1,5:1 à 4:1.

5. Procédé de mesure du segment antérieur de l'oeil d'un patient au moyen d'un dispositif selon la revendication 1, **caractérisé en ce que** plusieurs mesures interférométriques des surfaces optiques limites du segment antérieur de l'oeil sont effectuées, des valeurs de mesure se correspondant mutuellement étant détectées et un ensemble complet de valeurs de mesures étant calculé par ajustement sur des valeurs se correspondant respectivement mesurées lors de plus d'une mesure.

6. Procédé de mesure du segment antérieur de l'oeil selon la revendication 5, **caractérisé en ce que** la position relative de l'axe de l'oeil par rapport à l'axe optique du dispositif est déterminée et **en ce qu'**une direction des deux axes est obtenue par application d'une impulsion de fixation adaptée de manière correspondante au moyen du modulateur spatial de lumière.

7. Procédé de mesure du segment antérieur de l'oeil, notamment de la cornée, au moyen d'un dispositif selon la revendication 1, **caractérisé en ce qu'**en générant de manière définie des impulsions de focalisation sous des angles prédéterminés par rapport à l'axe optique du dispositif interférométrique, on réalise une mesure en des points prédéterminés de la cornée.

8. Procédé de mesure de l'amplitude d'accommodation au moyen d'un dispositif selon les revendications 1 et 2, **caractérisé en ce qu'**une région de décalage du modulateur spatial de lumière dans laquelle le patient peut voir de manière nette des symboles de test représentés sur celui-ci, peut être détectée.

9. Procédé de mesure du segment antérieur de l'oeil au moyen d'un dispositif selon les revendications 1 et 2, **caractérisé en ce que** pour l'adaptation à des défauts de vision éventuels du patient, le modulateur spatial de lumière est décalé de manière correspondante.

Fig. 1

Fig. 2

Fig. 3 (Messung 1)

Fig. 4 (Messung 2)

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19857001

- DE 10108797

**In der Beschreibung aufgeführte Nicht-Patentliteratur**

- **CH. HITZENBERGER.** Optical Measurement of the Axial Eye Length by Laser Doppler Interferometry. *Investigative Ophthalmology and Visual Science,* Marz 1991, vol. 32 (3), 616

- **DREXLER.** Submicrometer Precision Biometry of the Anterior Segment of the Human Eye. *Investigative Ophthalmology & Visual Science,* Juni 1997, vol. 38 (7), 1304